# EUROPEAN PATENT SPECIFICATION

(11) **EP 2 890 317 B1**
(45) Date of publication and mention of the grant of the patent: **13.12.2017**
(21) Application number: 13828270.2
(22) Date of filing: 09.08.2013
(51) Int. Cl.: A61B 18/00, A61B 18/20, A61B 18/18, A61N 5/00, A61N 5/067, A61B 17/00

(54) **APPARATUS FOR DERMATOLOGICAL TREATMENT**
VORRICHTUNG ZUR DERMATOLOGISCHEN BEHANDLUNG
APPAREIL DE TRAITEMENT DERMATOLOGIQUE

(30) Priority: 10.08.2012 US 201261681992 P
(43) Date of publication of application: 08.07.2015
(73) Proprietor: The General Hospital Corporation, Boston, MA 02114 (US)
(72) Inventor: MANSTEIN, Dieter, Coral Gables, Florida 33143 (US); KOSITRATNA, Garuna, Boston, Massachusetts 02144 (US)
(74) Representative: Maiwald Patentanwalts GmbH
(86) International application number: PCT/US2013/054362
(87) International publication number: WO 2014/026126

(56) References cited:
- WO-A1-2012/092057
- WO-A2-2007/134257
- WO-A2-2010/102197
- US-A- 5 520 679
- US-A- 5 839 446
- US-A- 6 059 772
- US-A1- 2009 105 696
- US-A1- 2009 137 996
- US-A1- 2010 168 724
- US-A1- 2011 098 691
- US-A1- 2011 098 789
- US-B1- 6 463 083
- US-B1- 6 482 199
- US-B1- 6 482 199
- US-B2- 7 635 362
- BENEDIKT JEAN AND THOMAS BENDE ED - SOROKINA ET AL: "Mid-IR Laser Applications in Medicine", 1 January 2003 (2003-01-01), SOLID-STATE MID-INFRARED LASER SOURCES; [TOPICS IN APPLIED PHYSICS], SPRINGER-VERLAG, BERLIN, GERMANY, PAGE(S) 530 - 565, XP008153048, ISBN: 978-3-540-00621-3 * abstract * * p 514; p 518, above; paragraph bridging pp 519-520; p 522, above; paragraph bridging pp 523-524; chapter 4.4 *
- CHRISTIANSEN, K ET AL.: 'Low density, non-ablative fractional C02 laser rejuvenation' LASERS SURG MED. vol. 40, no. 7, September 2008, pages 454 - 60, XP055198044

## Description

### TECHNICAL FIELD

The present disclosure relates to cosmetic methods and apparatus for fractional photothermolysis of skin and other tissues, in which a plurality of energy pulses having different properties can be provided onto a single location on the tissue, so as to form a hole therein, coagulate tissue within the hole, and then ablate at least a portion of the coagulated tissue.

### BACKGROUND Information

Fractional skin resurfacing relates to a cosmetic procedure where small regions of thermal damage are formed in skin tissue using electromagnetic energy, e.g. electromagnetic radiation (EMR), such as a laser beam. Each region is preferably small, e.g., less than I mm in diameter or less than 0.5 mm in diameter, and surrounded by substantially unaffected, healthy tissue. For example, the areal fraction of skin surface area covered by damaged tissue after a conventional fractional resurfacing treatment can be typically between about 5% and about 40-50%. Lower areal fractions can result in faster healing times and or less disruption of the epidermis, but the beneficial cosmetic effects may also be reduced because of the smaller volume of tissue damaged. Higher areal fractious can result in increased skin tightening, more rejuvenation and/or other desirable cosmetic effects, but healing times may be longer because of the larger amount of epidermal tissue that is thermally damage or removed.

The damaged regions of tissue can be generated by heating and/or ablation. Ablation can occur when the RMR is of sufriciently high intensity, sufficiently absorbed by the tissue, and applied in a sufficiently short time to vaporize a portion of the tissue, which can lead to formation of holes in the tissue and or removal of further tissue in an existing hole. If the energy is applied to the tissue with sufficient power intensity in a time that is shorter than the thermal relaxation time of the tissue, increased localised energy absorption and ablation of tissue can occur

Thermal damage in tissue can also be produced by EMR having a lower intensity and or longer pulse durations, such that the energy absorbed by the irradiated tissue is heated but not vaporized. Such milder thermal damage can "cook" the tissue, e.g., coagulate tissue and/or denature proteins such as collagen, which may result in local death of some cells and damage to other cells. EMR can also be delivered at intermediate times and intensities, such that both tissue ablation and significant coagulation occur m the irradiated tissue.

In dermatological applications, EMR generated by a laser is often provided in a form of one or more pulses, where the parameters associated with a pulse of EMR can include, e.g.. wavelength of the energy, total pulse energy (in J or mJ), beam width or diameter, and duration of a pulse. For example, the power of an EMR pulse can be determined as the total energy of the pulse divided by the duration of the pulse. Further, the intensity of a pulse can be determined as the power of a pulse divided by a cross-sectional area of the pulse beam. This intensity can be taken as an average intensity, because the energy and/or power may not be uniformly distributed over the temporal duration of the pulse and/or the area of the beam.

Different effects on tissue can be provided by EMR beams having differing properties, which is well-recognized in the art. For example, for a single laser source emitting EMR at a particular wavelength, one or more beam parameters can be varied to produce different effects in tissue. lf a beam pulse is provided with a particular total energy, reducing the pulse duration will result in a higher pulse power, and reducing the pulse duration and/or area or width of the pulse can result in a higher intensity. Similarly, the intensity of a pulse at a fixed beam diameter and duration (or pulse length) can be increased by increasing the total energy of the pulse.

Certain types of lasers such as, e.g., an excimer laser, may produce EMR that predominantly ablates biological tissue without generating significant heating or coagulation of nearby tissue, even when parameters of a pulse or beam are varied. Other types of laser sources. e.g., CO₂ lasers, Er:YAG lasers, or Nd:YAG lasers, can be used to ablate tissue if the EMR is provided at a sufficiently high intensity, and can also be adapted to heat or coagulate tissue with little andor no ablation when beam parameters are selected to generate pulses having lower intensities These lasers are commonly referred to as "ablative" lasers. In contrast, certain low-power laser sources or lasers emitting EMR at certain weakly-absorbed wavelengths such as, e.g.. some diode lasers, may be better suited for thermally damaging tissue and may not be capable of generating sufficient intensity of absorbed EMR to ablate tissue under typical operating conditions. Such lasers are often referred to as "non-ablative" lasers. Examples of such devices are described in WO 2007 134 257 and US 2009 137 996.

For example, EMR provided by a CO₂ laser at a wavelength of about 10.600 nm is well-absorbed by water, and can ablate skin tissue or other biological tissue when provided in a beam of sufficiently high intensity. The pulse duration and energy of a CO₂ laser can be varied, e.g., at a fixed beam width, to generate EMR that can ablate tissue, cause thermal damage in tissue without any ablation or vaporization, or to cause thermal damage with varying degrees of ablation. Exemplary parameters for various laser sources that can be used to generate ablation, coagulation, or a mix of both effects in tissue, including general relationships between such parameters, are known in the art for various types of lasers. For example, general parameter limits for producing ablation, coagulation with no ablation, or a mix of both effects in skin tissue using a CO₂ laser are described, e.g.. in U.S. Publication No. 2006/0149223 A1 by Hwang et al., and in U.S. Patent No. 6,159,204 issued to Hibst.

Regions of thermal damage can generate collagen shrinkage, coagulation, and/or a wound healing response that can lead to such effects as an overall tightening of the skin tissue and improved appearance in the treated area. Although some tightening of the skin occurs over time, it would be desirable to have fractional resurfacing methods and apparatus that facilitate faster healing times while generating a desirable rejuvenation effect in the treated skin tissue.

Accordingly, there may be a need to address and/or overcome at least some of the issues indicated herein above.

### SUMMERY OF EXEMPLARY EMBODIMENTS

According to exemplary embodiments of the present disclosure, method and apparatus can be provided for ablative fractional skin resurfacing that includes forming a small ablated hole (e.g., less than 1 mm in diameter, or less than about (0.5 mm) and then directing one or more further pulses into the hole sequentially to generate further tissue coagulation with little or no further tissue ablation, followed by one or more pulses to ablate a portion of the coagulated tissue. Such pulses can be generated using an ablative laser (e.g., a CO₂ laser, a CO laser, an erbium laser, for example, an Er: YAG or Er:YSGG laser, e.g., a CO₂ laser, a CO laser, an erbium laser such as an Er:YAG or Er:YSGG laser, and/or another type of YAG laser such as a Tm:YAG, Ho:YAG. or a Nd:YAG laser, or the like), and optionally by using an additional non-ablative laser. Parameters of the laser pulses can be selected and controlled using, e.g.. a control arrangement, to provide a plurality of pulses onto a single target location on the skin to achieve these effects.

For example, the energy pulses can be generally categorized as one of three general types. For example, a pulse of highly ablative electromagnetic radiation or energy (an "HA pulse") can be used, e.g.. to ablate a deep hole or to remove at least some previously-uncongulated tissue within or proximal to a hole, without generating any significant coagulation of adjacent tissue. A pulse of mildly or moderately ablative electromagnetic energy (an "MA pulse") can be configured generate a significant amount of non-ablative heating of adjacent tissue to produce further coagulation and/or thermal damage of tissue whit a relatively small amount of further tissue ablation, e.g., a portion of coagulated tissue that may be present within or proximal to an ablated hole. A pulse of non-ablative electromagnetic energy (an "NA pulse") can be configured to heat and coagulate tissue within or proximal to a bole with no associated tissue ablation or vaporization.

In certain exemplary embodiments of the present disclosure, a plurality of holes can be formed in the tissue, either simultaneously or sequentially using, e g.. methods and apparatus similar to those used for conventional ablative fractional resurfacing. A width or diameter of an ablative EMR beam used to ablate such holes can be less than 1 mm, e g., between about 0.1 mm and 0.5 mm. A hole formed by ablation using such a beam can have a width comparable to the beam width at the tissue surface, e.g.. less than about 1 mm or between about 0.1 mm and about 0.5 mm.

Individual holes can be further processed by directing one or more further pulses of energy into the hole. Various sequences of HA pulses, MA pulses, and/or NA pulses can be provided to generate particular effects in tissue. For example, after a deep hole is ablated using an HA or predominantly ablative MA pulse, one or more NA pulses and or predominantly coagulative NA pulses can then be directed into the hole to coagulate more tissue within the hole A portion of this coagulated tissue can then be ablated by directing one or more MA or HA pulses into the hole. This Exemplary procedure can optionally be repeated a number of times in a single hole to alternately form coagulated tissue within the hole and then remove at least a portion of it by ablation. Such exemplary pulse sequences may reduce healing or recovery times, for example, by directing further pulses of energy (EMR) into each ablated hole without a significantly increase in the hole depth or width. Rejuvenation effects (e.g. new collagen growth and or skin tightening associated with each such hole may be comparable or greater than that resulting from. e.g., a single ablative pulse having the same total energy as the sequence of pulses.

According to further exemplary embodiments of the present disclosure, a plurality of MA and/or NA pulses can be directed into the ablated hole after the ablative pulses to generate more coagulated tissue therein, such that the hole can be at least partially filled with coagulated tissue. In this exemplary manner, a larger amount of tissue removal and shrinkage can be generated in each hole without enlarging the hole dimensions, which may facilitate healing and/or reduce healing times, and can also lead to enhanced tissue rejuvenation effects for the procedure.

In additional Exemplary embodiments of the present disclosure, system and apparatus can be provided for directing pulsed electromagnetic energy onto biological tissue. Such exemplary system and apparatus can include a source of electromagnetic radiation (EMR) and a control arrangement, which can be configured to provide a plurality of pulses having predetermined parameters onto a single location of the tissue. Exemplary parameters that can be controlled for each pulse can include, e.g.. energy, duration, power, beam width, etc. Exemplary parameters that can be controlled for a plurality or sequence of pulses include, e.g., the number of pulses and intervals or delays between successive pulses. The control arrangement can include a microprocessor and or other processing arrangement, and can be configured to control the exemplary parameters associated with each pulse, as well as timing intervals or delays between successive pulses directed onto a single location in the tissue. The exemplary system and apparatus can further include an optimal arrangement configured to direct a plurality of pulses provided by the laser source(s) onto each of a plurality of particular locations on the tissue.

The exemplary system and apparatus can include one or more sources of electromagnetic energy, e.g., one or more lasers. For example, the apparatus can include two or more lasers, each capable or configured to produce EMR. at a different wavelength, e.g., an ablative laser and a non-ablative laser. In further embodiments, the system can include a single laser such as, e.g., a CO₂ laser or an erbium laser. Such single-laser systems can be configured to generate ablative, non-ablative, and/or partially ablative pulses based on the parameters selected for each pulse. For example, pulses having higher energies and/or power and/or shorter duration may be more ablative, whereas pulses provided at lower energy or power and/or longer durations can be non-ablative or only mildly ablative.

According to still further exemplary embodiments of the present disclosure, a method can be provided for directing pulsed electromagnetic energy onto biological tissue. The exemplary method can direct at least one ablative pulse of EMR onto a particular location on the tissue to ablate a hole therein, then direct at least one further pulse of EMR onto the same location, e.g.. into the ablated hole, to generate tissue coagulation therein, and then direct at least one further EMR pulse onto the same location to ablate at least a portion of the coagulated tissue formed. In additional exemplary embodiments of the present disclosure, the exemplary method can facilitate directing at least one further non-ablative or mildly ablative pulse of EMR onto the same location (e.g., into the hole) to coagulate further tissue in the hole and at least partially fill the hole.

These and other objects, features and advantages of the present disclosure will become apparent upon reading the following detailed description of exemplary embodiments of the present disclosure, when taken in conjunction with the appended drawings and claims.

### BRIEF DESCRIPTION OF THE DRAWINGS

Further objects, features and advantages of the present disclosure will become apparent from the following detailed description taken in conjunction with the accompanying figures showing illustrative embodiments, results and or features of the exemplary embodiments of the present disclosure, in which:
FIG. 1 shows two exemplary histograms of a section of skin tissue that has been exposed to five spatially-distinct laser pulses having the same total energy but different powers and durations:
FIGS. 2A-2D are Exemplary schematic diagrams illustrating the effects of ablative and non-ablative energy pulses on a hole made in biological tissue, in accordance with exemplary embodiments of the present disclosure
FIG. 3 is an Exemplary apparatus for producing particular sequences of laser pulses in accordance with embodiments of the present disclosure:
FIG. 4 shows two exemplary histograms of a section of skin tissue where different locations have each been exposed to a sequence of laser pulses, and the order of the sequences varied between locations; and
FIG. 5 is a bar graph of exemplary experimental observations of the effects of venous pulse sequences on irradiated skin tissue.

Throughout the drawings, the same reference numerals and characters, unless otherwise stated, are used to denote like features, elements, components, or portions of the illustrated embodiments. Similar features may thus be described by the same reference numerals, which indicate to the skilled reader that exchangers of features between different embodiments can be done unless otherwise explicitly stated. Moreover, while the present disclosure will now be described in detail with reference to the figures, it is done so in connection with the illustrative embodiments and is not limited by the particular embodiments illustrated in the figures. It is intended that changes and modifications can be made to the described embodiments without departing from the true scope of the present disclosure as defined by the appended claims. The invention is defined in the claims, other embodiments being merely exemplary.

### DETAILLED DESCRIPTION OF THE PREFERRED EMBODIMENTS

According to exemplary embodiments of the present disclosure, method and apparatus can be provided for directing energy onto biological tissue, e.g., for fractional skin resurfacing, that includes ablating a plurality of spaced-apart holes over a region of the tissue surface using electromagnetic radiation (EMR) or energy, such as optical energy produced by a laser, and then directing one or more further pulses of EMR having different parameters into at least some of the holes to generate coagulated tissue therein and or ablate at least a portion of the coagulated tissue that has formed therein. Further pulses can be directed into the same hole to coagulate and/or remove additional tissue from the holes. Further coagulation can be generated using pulses generated by the ablative energy source, optionally at a lower power level and/or longer pulse duration, and or by a further source of electromagnetic energy that is non-ablative.

The energy pulses can be generally categorized by thee typical effects when directed onto biological tissue such as skin. For example, a highly ablative electromagnetic energy pulse (an "HA pulse") can vaporize tissue and thereby ablate a deep hole, e.g., to remove at least some previously-uncoagulated tissue, and not for a significant amount of coagulation in adjacent tissue. A mildly ablative electromagnetic energy pulse (an "MA pulse") can ablate tissue. e.g., a portion of coagulated tissue previously formed within or proximal to an ablated hole, while also being absorbed sufficiently by adjacent tissue to generate additional coagulation. A non-ablative electromagnetic energy pulse (an "NA pulse") can heat tissue locally to coagulated a portion of it within or proximal to a hole, with no associated tissue ablation or vaporization.

Exemplary lasers that can be used in embodiments of the present disclosure include, e.g.. a CO₂ laser, a CO laser, an erbium laser such as an Er:YAG or Er:YSGG laser, a Tm:YAG or Ho:YAG laser, or the like. The exemplary laser can be configured to provide EMR in one or more pulses In certain Exemplary embodiments of the present disclosure, a single laser source such as the ones listed in this paragraph can be used, where the laser can be controlled to provide pulses of EMR that can ablate or vaporize skin tissue, and further pulses of EMR having properties that can cause thermal damage in tissue without ablating it. According to further exemplary embodiments of the present disclosure, at least two laser sources can be used, where at least one source is capable of providing pulsed energy that can ablate skin tissue, and a second laser source that is capable of providing EMR pulses that cause thermal damage in skin tissue without ablating such tissue.

The exemplary characteristics of an electromagnetic energy pulse (HA, MA. NA) can be determined by several parameters or combinations thereof including, for example, the wavelength(s) of the radiation (which can affect the absorption of energy by the tissue), the type of tissue, the total energy or power of the pulse, the beam diameter, pulse length/duration, and the tissue temperature (e.g., pre-heating). For example, for particular total pulse energy, decreasing the pulse duration and/or beam width can lead to a higher local power or energy intensity, which may tend to produce more ablation of tissue. In contrast, increasing the pulse duration and or width for a particular total pulse energy can decrease the pulse power and intensity, which can lead to more heating or coagulation of tissue with little or no removal of tissue by ablation

As an example, for a CO₂, laser emitting pulses of EMR having a beam width less than 1 mm in diameter (e.g., between about 0.1 mm and 0.5 mm), an HA pulse can have a total energy of between about 5 mJ and 500 mJ, and duration between about 0.05 ms and I ms, with longer pulse durations corresponding to higher pulse energies (for a particular beam width) to provide sufficient power intensity to primarily ablate the tissue. Similarly. A pulses can have pulse energy between about 5 mJ and 500 mJ, and longer pulse duration between about 0.5 ms and 100 ms, with higher pulse energies corresponding to longer pulse durations to reduce the effective power and intensity, thereby avoiding vaporization of tissue. MA pulses can have properties with intermediate parameter values in these exemplary ranges provided for HA and NA pulses, e.g.. similar pulse energies and somewhat longer pulse durations than HA pulses, e.g., pulse durations up to about 5 ms or 10 ms, or even longer at pulse energies in the higher end of the exemplary HA range, which may still be used to ablate tissue.

As a further example, for an Er:YAG laser emitting pulses of EMR having a beam width less than 1 mm in diameter (e g., between about 0.1 mm and 0.5 mm), an HA pulse can have a total energy of between about 100 mJ and 1000 mJ, and duration between about 0.1 ms and 2 ms, with longer pulse durations corresponding to higher pulse energies to provide sufficient power intensity to primarily ablate the tissue. Similarly, NA pulses can have pulse energy between about 10 mJ and 500 mJ, and pulse duration between about 5 ms and 200 ms, with higher pulse energies corresponding to longer pulse durations to avoid vaporization of tissue. Again, MA pulses can have properties with intermediate parameter values in these exemplary ranges provided for HA and NA pulses, or somewhat longer pulse durations than HA pulses for similar pulse energies, e.g., pulse durations up to about 20 ms or 50 ms, or even longer at pulse energies in the higher end of the exemplary HA range, which may still be used to ablate tissue.

In general, for a particular beam width and laser emitting EMR at a particular wavelength (and thus a particular absorption value in skin tissue), shorter pulse durations and higher pulse energies will result in a higher pulse power and intensity (for a particular beam width or diameter), and tend to be more ablative. Conversely, longer pulse durations with lower pulse energies tend to be non-ablative or less ablative, producing more thermal damage in the tissue while vaporizing little or no tissue. Because the ablative/no-ablative characteristics or a pulse depend on a plurality of factors, such as pulse energy, duration and width (at a particular wavelength having a particular absorption coefficient in tissue), ranges of parameter values for HA, MA and NA beams can overlap, as indicated above. For example, a 500 mJ pulse from a CO₂ laser that is 0.5 mm wide can be ablative if the pulse duration is short, on the order of a few milliseconds, and it can be mildly ablative or non-ablative if the pulse duration is longer, e g., a few hundred milliseconds.

Specific parameters for a laser emitting EMR at a particular wavelength, which correspond to HA. NA, or MA pulses, are known in the art and/or can be readily determined by simple calculations based on the known absorptivity of the EMR at the emitted wavelength and the known vaporization enthalpy and thermal relaxation time of skin tissue, for a particular beam width or diameter. If multiple pulses of EMR are provided in succession, with short intervals between them, the pulse energy and/or duration may be decreased somewhat on later pulses to account for local preheating effects in the tissue, which can lower the power intensity to ablate tissue.

Further, certain lasers emitting EMR at a wavelength having a relatively low absorption coefficient in skin tissue may not be capable of ablating biological tissue because of limitations on pulse energies and pulse durations. Such non-ablative lasers can, e.g., be used with exemplary embodiments of the present disclosure to provide NA pulses.

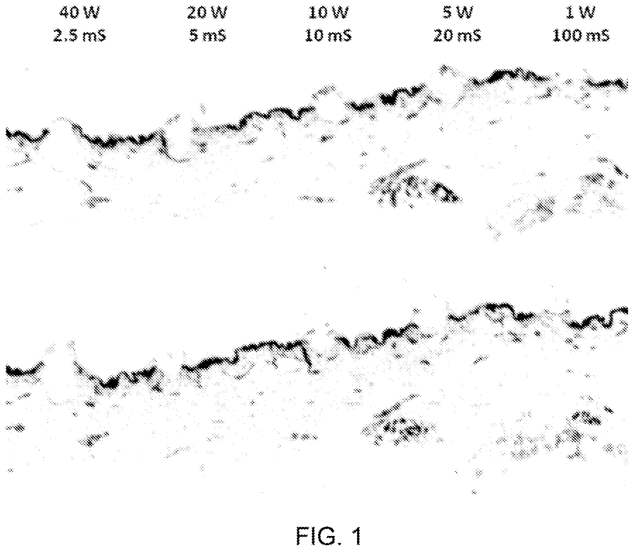

Two cross-sectional exemplary images of a histological sample showing the effects of several exemplary laser pulses on skin tissue are shown in FIG. 1 The five spatially distinct pulses were generated by a CO₂ laser. The total energy of each pulse was set to 100 mJ, and each pulse had a power density of 25 W/cm² and a fluence or 2.5 J cm² (based on the beam width or spot size). However, the power and duration of each pulse were varied, as indicated by the parameter labels above each pulse location. The leftmost pulse had the highest power level tested. 40 W, corresponding to the shortest pulse duration of 2.5 ms. The rightmost pulse had the lowest energy, 1 W, and the corresponding largest duration of 100 ms. The three intermediate pulses had (left to right) a pulse power of 20 W, 10 W, and 5 W, and corresponding pulse durations or 5 ms, 10 ms, and 20 ms, respectively.

As can be seen in FIG. 1, the pulse with the highest energy and shortest duration ablated the deepest hole in the tissue. As pulse power decreases left to right, the corresponding depths of the ablated holes become shallower. The rightmost pulse barely ablated any tissue, and instead coagulated a greater volume of tissue. In terms of the nomenclature used herein, the 40 W/2.5 ms pulse and the 20 W/5 ms pulse may be considered to be moderately ablative (MA) pulses that predominantly ablate tissue with a relatively small amount of associated coagulation or thermal damage. The 10 W/10 ms pulses and the 5 W/20 ms pulse may be considered to be mildly ablative (MA) pulses. The 1 W/100 ms pulse may be considered to be a substantially non-ablative (NA) pulse.

The exemplary results shown in FIG. 1 illustrate that a single ablative laser can be used to generate these different types of pulses by varying the parameters associated with them, such as pulse power and pulse duration. Each of the pulses directed onto the tissue in FIG. 1 had the same total energy of 100 mJ. However, other parameters such as, e.g., the spot size and/or energy of individual pulses can also be varied to produce particular degrees of ablation and or coagulation, when they interact with tissue.

According to certain exemplary embodiments of the present disclosure, methods and apparatus can be provided for ablative fractional skin resurfacing that includes forming a small ablated hole using an EMR beam having a width that is, e.g.. less than 1 mm in diameter, or between about 0.1 mm and 0.5 mm, which can ablate holes in the tissue having comparable widths. The EMR beam can comprise one or more HA pulses and/or one or more MA pulses, e.g.. mildly or moderately-ablative pulses that ablate sufficient tissue to generate a hole that extends at least into the dermis. Each of the ablated holes can extend from the skin surface through the epidermal layer and to at least a particular depth within the dermis. The fraction of the skin surface in a treatment area that is covered by such holes can be, e.g., between about 0.05 and 0.5 (5-50%), for example, between 0.1 and 0.4 (10-40%). These exemplary ranges of hole sizes and surface areal coverage can be sufficiently small to allow each damaged region to be surrounded by healthy tissue, which can speed healing and reduce or avoid an appearance of visible markings, while being large enough to generate a cosmetically desirable effect such as rejuvenation, shrinkage, wrinkle reduction, and/or a reduction in appearance of pigmented defects.

After the ablated holes are formed, one or more further pulses can be directed onto the same location as the ablative beam, e.g., into the ablated holes, to generate further tissue coagulation and thermal damage. Such pulses can be generated using an ablative laser (e.g., a CO₂ laser or an erbium laser) with parameters selected to produce pulses with lower power and/or intensity (e.g., MA or NA pulses). In certain exemplary embodiments of the present disclosure, the further pulses can be provided by a second laser source that produces EMR having a different wavelength, e.g.. a non-ablative laser.

Exemplary parameters of the laser pulses can be selected and controlled using, e.g.. a control arrangement, to provide pulses having various characteristics as described below. In general, energy pulses having higher power and shorter duration tend to more strongly ablate tissue, vaporizing a portion thereof based on the large amount of energy that is absorbed by the tissue in a short time (e.g., shorter than a local thermal relaxation period). For such ablative pulse, the energy tends to heat up tissue quickly enough to vaporize a portion thereof. Such highly ablative pulses tend to have a very thin thermal affected zone around the ablated volume. In contrast, pulses having lower power and longer durations tend to heat up tissue more slowly, such that some energy can be dissipated to surrounding tissue and generate more tissue coagulation, protein denaturation, etc. A single pulse or certain types of EMR (e.g. EMR having a wavelength within certain ranges known in the art) can both ablate and coagulate tissue, with the relative amount of ablation and coagulation depending on the various parameter associated with the energy pulse. A laser pulse having a particular set of parameters may also affect different tissues differently, e.g., based on the chromophores present, tissue structures present in the tissue, etc. For example, the energy generated by a CO₂ laser tends to be strongly absorbed by water and thus its effect on tissue, may depend more on the amount of water present in the tissue than on the presence or absence of visible chromophores such as pigments.

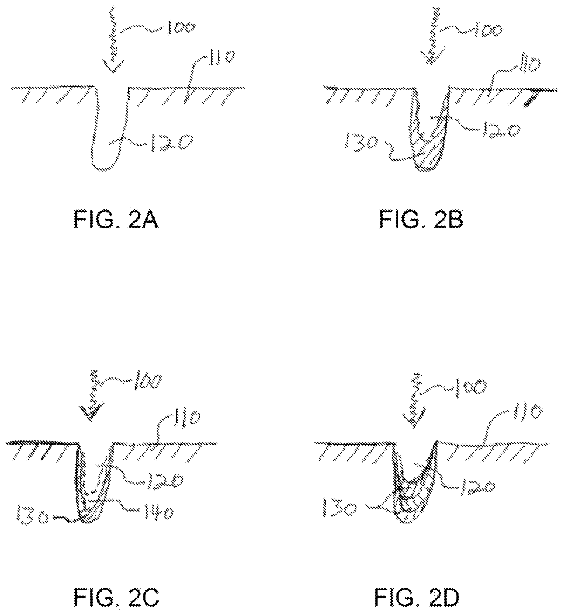

As shown in FIG. 2A, a beam 100 of ablative energy (e.g., one or more HA or MA pulses) can be directed onto a particular location on the surface of tissue 110 (e.g., skin) to form a hole 120 therein, according to an exemplary embodiment of the present disclosure FIG. 2B shows a zone of coagulated tissue 130 that can be formed around a hole 120 that was ablated in tissue using one or more HA or MA pulses. In certain exemplary embodiments, such coagulated tissue 130 can be formed (or the amount of it increased) by directing one or more further pulses 100 of NA energy onto the same location and into the hole 120 as shown, e.g., in FIG. 2B.

The coagulation tends to contract and/or shrink tissue along portions of the hole, which may contain collagen or other structural proteins. Additional tissue adjacent to the coagulated tissue 130 may also be heated to a lesser degree and respond to the applied energy, such that coagulated tissue 130 may partially refill the lower portion of die hole 120. The time for coagulation to occur after exposure of tissue to an energy beam 100 (e.g. a laser) can be very short, for example, on the order of milliseconds or tens of milliseconds.

For example, one or more further laser pulses 100, e.g.. HA pulses, can be directed onto the same location (e.g., into the hole 120), as shown in FIG. 2C, to ablate at least a portion 140 of the coagulated tissue130 that has formed within the hole 120. In FIG. 2C, the boundary of the coagulated tissue 140 that was removed is indicated by the dashed line. This procedure facilitates enhanced tissue removal and a greater degree of shrinkage within a single ablated micro-hole 120 by directing a plurality of energy pulses 100 into the hole 120. Such funher tissue coagulation and removal can be achieved, e.g.. without enlarging the size of the hole 120 at the surface of the tissue 110.

The exemplary procedure illustrated in FIGS. 2A-2C, e.g., tissue ablation to form a hole 120, followed by coagulation of tissue in the hole 120 and subsequent ablation of some of the coagulated tissue 130 can facilitate ablation of more subsurface tissue without deepening the hole 120. The exemplary procedures shown in FIG. 2B and 2C, i.e., forming coagulated tissue 130 in the hole 120 and then ablating at least some of this coagulated tissue 130 can be repeated to draw further tissue 110 into the lower portion of the hole 120 and then ablating more of it, thereby removing more tissue 110 that is proximal to lower portions of the hole 120 without deepening the hole significantly. In contrast, providing stronger or a larger number of only ablative pulses 100 of EMR would tend to deepen the hole 120 while ablating further tissue

The exemplary coagulative and/or ablative procedures illustrated in FIGS. 2B and 2C, respectively, can be conducted with one or a plurality of EMR pulses 100. For example, the tissue coagulation procedure shown in FIG. 2B can be achieved by directing either one or a plurality of pulses 100 of NA and/or MA energy into the hole 120. If a pulse 100 of MA energy is used, it can predominantly coagulate tissue to with only a moderate amount of ablation, e.g., such that the volume of coagulated tissue 130 increases after the tissue is irradiated. Similarly, the tissue ablation shown in FIG. 2C can be achieved by directing either one or a plurality of pulses 100 of HA and/or MA energy into the hole 120. If a pulse 100 of MA energy, is used, it can predominantly ablate tissue 110 with a relatively small amount of coagulation, e.g., such that the volume of coagulated tissue 130 decreases after the tissue 110 in the hole 120 is irradiated.

In further exemplary embodiments of the present disclosure, one or more non-ablative energy pulses 100 can be directed into the hole 120. e.g, after some coagulated tissue 130 is ablated as shown in FIG. 2C. For example, one or more non-ablative (NA) pulses 100 of EMR can be directed into the hole 120 to at least partially "fill" the hole with coagulated tissue 130, as shown in FIG. 2D. In further exemplary embodiments, coagulated tissue 130 can be generated within the hole by directing one or more predominantly non-ablative pulses 100 (e.g., MA pulses) pulses into the hole 120, where the properties of the MA pulses can be selected such that additional tissue is coagulated with a small or negligible amount of such tissue being ablated. In still further exemplary embodiments of the present disclosure, a plurality of MA and NA pulses 100 can be directed into the hole 120 to "fill" it with coagulated tissue 130. In such exemplary procedures, the depth of the hole 120 can be decreased, by forming additional coagulated tissue 130 (as shown in FIG. 2D) after forming and ablating at least a portion of such coagulated tissue 130 in an ablated hole 120 (as shown in FIGS. 2B and 2C. Such partial "filling" of the hole 120 after forming a small hole 120 and then generating and removing some coagulated tissue 130 therein, can facilitate faster healing times while also providing a greater amount of tissue damage associated with the hole 120 as compared, e.g., to the healing time and extent of tissue damage associated with just ablating a hole 120 as shown, e.g., in FIG. 2A.

The exemplary interval between successive pulses 100 can be determined based on several factors. For example, certain ones of the pulses can be delivered with substantially no interval between them. In further exemplary embodiments of the present disclosure, an interval between certain pulses 100 can be provided e.g.. after an NA or MA pulse, to allow coagulation of tissue 110 to occur before irradiation of the tissue with a subsequent pulse. Such coagulation, can form in several to tens of milliseconds, e.g., based on considerations of local thermal relaxation times. Accordingly, intervals between successive pulses 100 applied to the same location can be, for example, tens of milliseconds, e.g., 10-30 ms or more, up to 100 ms or greater. Relatively smaller intervals may lead to more local preheating as the tissue 110 may have less time to cool off between pulses 100. This cumulative preheating can be accounted for, e.g.. to generate increased coagulation, and/or ablation with subsequent pulses 100. that have the same or reduced intensity, power, etc. Setting appropriate exemplary pulse intervals can also vary the relative amounts of coagulation and ablation that are generated, e.g.. by a plurality of MA pulses having constant pulse properties.

In still further exemplary embodiments of the present disclosure, various combinations of beam parameters can be used to achieve improved cosmetic effects. For example, a wider non-ablative beam diameter can be used to generate coagulated tissue 130 along the hole sides, and then a narrower ablative beam pulse 100 can be applied to ablate a portion of this tissue. Other parameters, such as beam pulse energy, pulse duration, interval between pulses, etc. can be varied to generate desirable patterns of coagulation and tissue removal. For example, intervals between successive pulses 100 can be selected to facilitate formation and shrinkage of coagulated tissue 130, followed by ablation of a portion of such tissue.

In certain exemplary embodiments of the present disclosure, the positioning of different beam pulses 100 can be shifted slightly relative to the hole center, which may generate more coagulated tissue 130 along the side walls of the micro-holes 120 by increasing the amount of energy directed onto the hole sides. The shape of the beam cross-sections can also be varied; for example, a beam 100 (ablative and/or non-ablative) having an ovoid or linear cross-section shape can be used. The long axis of such an elongated beam cross-section shape can be rotated, e.g.. between pulses 100, to generate additional coagulation and or ablation along the sides of the micro-holes 120.

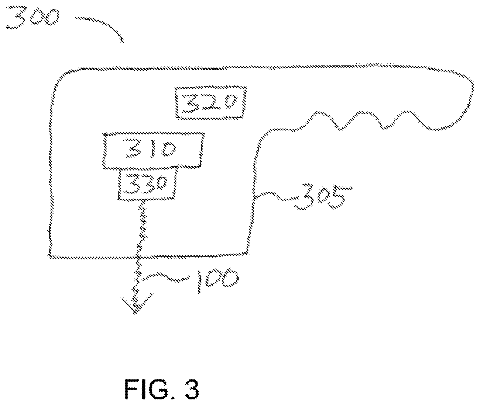

An exemplary apparatus 300 that can be used to perform cosmetic fractional resurfacing of skin tissue in accordance with certain exemplary embodiments of the present disclosure is shown in Fig. 3. The exemplary apparatus 300 can include an EMR source 310, a control arrangement, 320 provided in communication with the EMR source 310, and an optical arrangement 330. One or more of these components can be provided in a housing 305, e.g., as shown in FIG. 3. The housing 305 can be provided in a shape of a handpiece, or in another form that can enclose one or more of the components shown in FIG. 3. A power source (not shown) configured or adapted to provide electrical power to the EMR source 310 and/or the control arrangement 330 can also be provided, e.g., within or external to the housing 305.

The EMR source 310 can include at least one source of electromagnetic radiation (e.g., optical radiation), such as a laser. For example, the EMR source 310 can include a CO₂ laser or another type of laser, such as an erbium laser. In certain embodiments, the ER source 310 can include a plurality of lasers. For example, the EMR source 310 can include an ablative laser and a non-ablative laser. In certain exemplary embodiments of the present disclosure, the EMR source 310 can be provided external to the housing 305, and electromagnetic energy provided by the EMR source 310 can be directed to the optical arrangement 330, e.g., using one or more waveguides or the like, such as one or more optical fibers, a hollow fiber waveguide, etc.

The optical arrangement 330 can include, e.g.. one or more lenses, waveguide, reflectors, translators, motors and or beam splitters. It can be configured to direct EMR from the EMR source 330 onto at least one particular location, e.g., to direct a plurality of EMR pulses 100 as described herein onto a particular location on a skin tissue that is positioned relative to the apparatus 300. The optical arrangement 330 can be further configured to direct such pluralities of EMR pulses 100 onto a plurality of locations, e g., to form a plurality of holes 120 in a target region of tissue, with further pulses 100 directed into each hole 120 to coagulate and further ablate tissue within the hole 120 as described herein. Such holes 120 can be formed on a target region of tissue 110, e.g., as a plurality of spaced-apart holes 120 with healthy or substantially undamaged tissue between them, as can be done in conventional fractional resurfacing procedures.

The control arrangement 320 can be specifically configured to control and/or adjust properties of the EMR source 310 to provide predetermined pulse sequences of EMR as described herein. For example, the control arrangement 320 can be configured or adapted to control or adjust properties of one or more pulses 100 of EMR to form an ablated hole 120 in tissue 110, as shown in FIG. 2A, then control the EMR source 310 to direct one or more further pulses 100 of EMR having different properties into the hole 120 to coagulated tissue therein as shown, e.g., in FIG. 2B, and then control the EMR source 310 to direct one or more still further pulses 100 of EMR having particular properties into the hole 120 to ablate a portion of the coagulated tissue 130 as shown, e.g., in FIG. 2C. Optionally, the EMR source 310 can also be controlled by the control arrangement 320 to then direct one or more coagulative beams or pulses 100 (e.g., MA or NA pulses 100) into the hole 120 to further coagulate tissue therein, as shown in FIG. 2D.

Exemplary properties of the energy beams or pulses 100 produced by the EMR source 310 that can be controlled or adjusted by the control arrangement 330 can include, e.g., a focal diameter, a pulse duration, a pulse rate, a total pulse energy, a pulse power, a number of pulses, a frequency of pulses 100 (or, alternatively, an interval between successive pulses 100), and/or a pulse intensity. Some of these parameters can be related, e.g., the pulse energy divided by the pulse duration can determine the pulse power, the power and beam width or diameter can determine the pulse intensity, etc.

For example, a smaller focal diameter can generate a deeper ablated hole 120 for a particular pulse energy and duration, although there may be additional spreading of the beam 100 deeper in the tissue 110. Similarly, pulse duration can be varied for a fixed total pulse energy and beam width to control the corresponding power and intensity.

In further exemplary embodiments of the present disclosure, the EMR source 310 can comprise two or more lasers or other components capable of providing EMR, and the control arrangement 320 can be configured or adapted to control the timing and parameters of EMR provided by each of the lasers. The two or more lasers, if present, can be selected to provide EMR at different wavelengths. For example, one laser can provide EMR at wavelengths that typically ablate biological tissue, whereas a further laser can provide EMR at a wavelength that is more weakly absorbed by tissue and can generally heat irradiated tissue without ablating it.

In still further exemplary embodiments of the present disclosure, the control arrangement 320 can be configured or adapted to control the optical arrangement 330, e.g., to direct EMR pulses 100 from the EMR source 310 onto one or more particular locations. Such control can be adapted, for example, to provide a plurality of pulses onto a plurality of particular locations, where the pulses 100 are controlled to perform the exemplary sequence of processes illustrated in FIGS. 2A-2C or 2A-2D and described herein at each location.

The exemplary apparatus and methods described herein can. e.g., generate a greater degree of shrinkage around an ablated micro-hole as a greater amount of coagulated tissue is formed around each hole (with portions thereof being ablated) as compared, for example, to an ablated hole formed using a single pulse of energy or several pulses of ablative energy, with no significant coagulation being produced and then ablated as described herein.

Exemplary pulse sequences having a larger number of pulses 100 can be directed onto a single location to ablate more tissue within the same hole 120 and generate further tissue coagulation. This can lead to greater overall tissue shrinkage and/or wrinkle removal as compared to that which may be generated by the same number of ablated surface spots in a conventional ablative fractional resurfacing procedure. Additionally, an ablated hole 120 may be filled to a substantial degree by increasing the tissue volume within the initial hole 120 (with coagulative pulses such that the volume increase from coagulation is greater than the volume removed by ablative pulses.

In a manner similar to that used in conventional fractional resurfacing procedures, a plurality of holes 120 can be formed in a target area of skin, and then treated with a plurality of EMR pulses 100 as described herein. A local areal fraction of the skin surface covered by the holes can be, e.g., between about 0.05 and about 0.5. Further holes 120 can be formed in regions proximal to the treated area, and a plurality of pulses 100 directed into these additional holes 120. In certain exemplary embodiments of the present disclosure, a plurality of pulses 100 as described herein can be directed onto a single location on the skin, e.g., to form a hole 120, coagulate tissue therein, and ablate at least a portion of the coagulated tissue 130 as shown, e.g., in FIGS. 2A-2C. Optionally, further pulses 100 can then be directed into the hole 120 to coagulate further tissue and fill at least a portion of the hole 120 before performing the same or similar procedure to form and irradiate a second hole 120. The procedure can be repeated until the entire target area has been treated. Geometrical parameters for initial ablative hole sizes and hole patterns/spacings that are used in conventional ablative fractional resurfacing procedures may also be used in additional exemplary embodiments of the present disclosure.

### Example 1

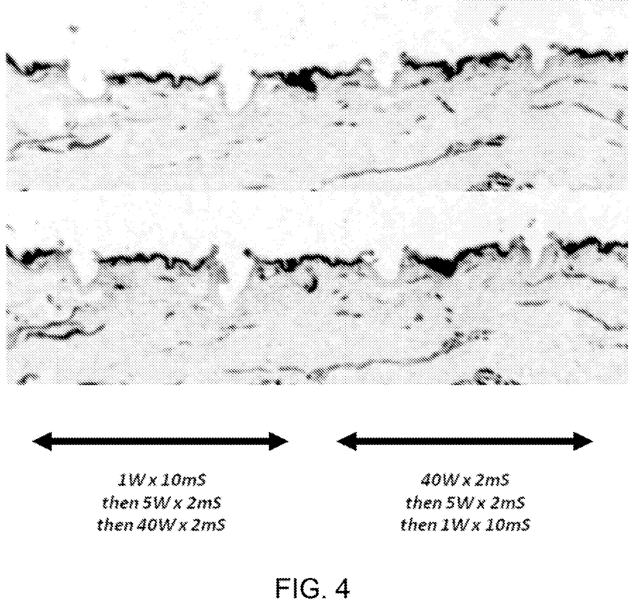

Two cross-sectional images of a histological sample showing the effects of two exemplary laser pulse sequences on *ex vivo* skin tissue are shown in FIG. 4. The skin sample was irradiated with pulses generated by a CO₂ laser. The holes shown in FIG. 3 were generated by sequences of three pulses having the powers and durations indicated below the cross-sectional images. For example, the two rightmost holes shown in FIG. 3 were formed by first directing an HA pulse onto the tissue (40 W, 2 ms duration), followed by an MA pulse (5 W, 2 ms duration), and finally an NA pulse (1 W, 10 ms duration) onto the same location. The time interval between pulses was about 10 seconds, although much shorter pulse intervals can be used.

Similarly, the two leftmost holes shown in FIG. 4 were generated by the pulse sequence denoted below these holes. First, an NA pulse with a power of 1 W and duration of 10 ms was directed onto the tissue. Next, an MA pulse having 5 W power and 2 ms duration was directed onto the same location Finally, an HA pulse having a higher power of 40 W and a duration of 2 ms was directed onto the same spot.

The holes on the left side of FIG. 4 appear deeper than the holes on the right. This can be attributed to the sequence of pulses, in which the final pulse is an HA pulse that would tend to deepen the hole and ablate some of the coagulated tissue that may be present therein. In contrast, the two holes on the right of FIG. 4 appear somewhat shallower, with a larger coagulated zone visible around these holes. The HA pulse in the two holes on the right was applied first, followed by an MA pulse and then an NA pulse. The MA pulse would ablate a smaller volume of tissue and the NA pulse would ablate little if any further tissue while generating more coagulation, thus leading to a shallower hole with more coagulated tissue surrounding it.

The effects of the two exemplary pulse sequences shown in FIG. 4 suggest that the size and coagulation geometry of a hole formed in tissue can be altered by the sequential properties of the applied pulses. For example, characteristics of the resulting hole and amount of residual coagulated tissue can be varied, e.g.. by changing the order of a plurality of pulses having different parameters. Accordingly, pulse sequences in accordance with exemplary embodiments of the present disclosure described herein, which include, for example, at least one ablative pulse to form a hole in tissue followed by at least one non-ablative or mildly ablative pulse to coagulate tissue in the hole, and then at least one ablative pulse to remove at least some of the coagulated tissue, can produce desirable effects that are different from an application of similar pulses in a different order

### Example 2

A Lumenis UltraPulse system with AcuScan 120 handpiece (Lumenis Surgical) that includes a controllable CO₂ laser was modified with a controller arrangement. The controller arrangement facilitated programming and control of particular pulse sequences in accordance with exemplary embodiments of the present disclosure. 10 sequences of energy pulses (A2-J2) were directed into different locations on a 2 cm x 2 cm sample of previously-frozen human abdominal skin.

The first three sequences (A2-C2) included only ablative 60 W pulses, with a total energy of 100 mJ per sequence. Pulse energies and durations for these ablative sequences are specified in Table 1 below. The exemplary system was programmed in accordance with embodiments of the present disclosure to irradiate the tissue with five further sequences of pulses (D2-H2), with a different location being irradiated by each pulse sequence. Pulse energies and durations for these five sequences, which include a mix of 60 W pulses that were primarily ablative (HA/MA) and 1 W (NA) pulses are also specified in Table 1 bellow. The total energy of each of these five sequences that was directed onto the *ex vivo* skin tissue was also 100 mJ. Finally, two further ablative pulse sequences (I2-J2), each with a total energy of 50 mJ as specified in Table 1 bellow, were also directed onto distinct locations on the skin sample.

Pre- and post- exposure pictures of the irradiated tissue were obtained using a Nikon D80 camera with AF Macro Nilkor 60mm lens. The irradiated skin samples were frozen and sectioned, and then stained with the NBTC stain. The lesion sizes (i.e. width and depth of both the ablated hole and the coagulation zone) were measured under light transmitted microscope (Olympus BH-2).

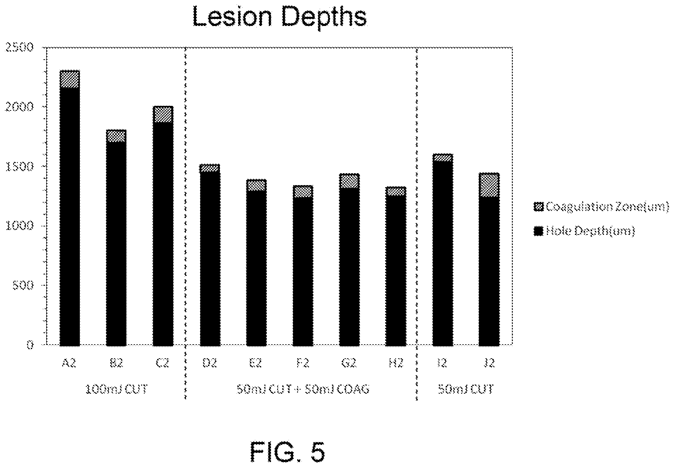

Observed ablated hole depths and coagulated tissue depth (at the bottom of each hole) are shown in FIG. 5 for each pulse sequence A2-J2. These exemplary pulse sequences can be grouped into three main categories. Pulse sequences A2, B2 and C2 each provide a total of 100 mJ of HA energy with differing pulse durations and intervals. Each of pulse sequences D2-G2 delivers a total of 100 mJ of HA energy with different sequences and values for pulse power, durations, and intervals between pulses. Pulse sequences H2 and 12 each provide a total of 50 mJ of HA energy with differing pulse durations and intervals. These different types of pulse sequences are labeled in FIG. 5 and separated by vertical dashed lines.

The purely ablative 100 mJ pulse sequences A2-C2 resulted in the deepest holes, with some coagulated tissue observed at the bottom of the holes. The sequences B2 and C2, where the ablative energy was divided into 5 and 10 pulses, respectively, resulted in somewhat shallower holes than sequence A1, where the 100 mJ was delivered in a single energy pulse. This may result from a small degree of tissue coagulation that may occur between the 60 W pulses followed by subsequent ablation of some of this coagulated tissue. In contrast, the single 100 mJ ablative pulse of A1 could ablate a deeper hole before any coagulation could occur.

Pulse sequences D2-H2 include different sequences of non-ablative 1 W pulses and 60 W pulses that are predominantly ablative (e.g., HA or MA pulses). In general, these "mixed" pulse sequences with 100 mJ total energy result in shallower hole depths than those resulting from ablative pulse sequences A2-C2, each of which also had a total energy of 100 mJ. The hole depths resulting from pulse sequences D2-H2 are also shallower than those resulting from the pulse sequences 12 and J2, each of which only delivered a total of 50 mJ of energy (half as much) into the tissue. The sallower holes resulting from the "mixed" pulse sequences may, e.g., facilitate faster healing times when formed as part of a fractional resurfacing procedure as compared to deeper holes.

Pulse sequences E2, F2, and G2 include at least one exemplary coagulative pulse (1 W) that is preceded by and followed by at least one exemplary ablative pulse (60 W). Pulse sequence D2 includes one coagulative (NA) pulse followed by five ablative (HAMA) pulses, and pulse sequence G2 includes five ablative (HA/MA) pulses followed by one coagulative (NA) pulse. Although not conclusive, it has been observed that the hole depths resulting from pulse sequences E2, F2 and H2, which are in accordance with exemplary embodiments of the present disclosure, are slightly shallower than the hole depths observed for pulse sequences D2 and G2.

The total volume of tissue removed by the various pulse sequences could not be measured directly in this study, because of the integrated effects of the energy pulses such as tissue ablation and coagulation. Nevertheless, it is likely that a greater amount of tissue was removed by embodiments of the present disclosure as expressed in the exemplary pulse sequences D2, E2. F2 and H2 than by the other pulse sequences, because of the presence of ablative pulses following coagulative or non-ablative pulses. The resulting hole depths of these exemplary pulse sequences are still somewhat shallower than the hole depths observed by other pulse sequences tested. The hole depth could be reduced further, e g., by using pulse sequences that include further non-ablative pulses at the end of the sequence, e.g., to provide a "hole-filling" effect such as that illustrated in FIG. 2D.

A general shrinkage of the irradiated *ex vivo* tissue samples was observed for the various pulse sequences tested. Such shrinkage can result from. e.g, tissue coagulation and denaturation of some tissue components such as collage. However, the desirable cosmetic effects of fractional resurfacing procedures in living tissue (e.g. skin tightening or wrinkle reduction) result from stimulated healing responses in the tissue as well as direct thermal effects on the tissue components. Accordingly, although not directly observed, the enhanced thermal damage and tissue removal, together with shallower hole depths, provided by embodiments of the present disclosure may likely lead to more effective skin tightening and/or faster healing times as compared to conventional resurfacing procedures performed using comparable energy and geometry parameters, e.g., those that deliver the same amount of energy to ablate holes in the tissue at the same surface coverage.

**TABLE 1. Exemplary Ablative and Non-Ablative Pulse Sequences**

| | *Pulse sequence:* | *1* | *2* | *3* | *4* | *5* | *6* | *7* | *8* | *9* | *10* |
|---|---|---|---|---|---|---|---|---|---|---|---|
| **A2** | Power (W) | 60 | | | | | | | | | |
| | Pulse duration (ms) | 1.67 | | | | | | | | | |
| | Energy (mJ) | 100 | | | | | | | | | |
| **B2** | Power (W) | 60 | 0 | 60 | 0 | 60 | 0 | 60 | 0 | 60 | |
| | Pulse duration (ms) | 0.33 | 50 | 0.33 | 50 | 0.33 | 50 | 0.33 | 50 | 0.33 | |
| | Energy (mJ) | 20 | 0 | 20 | 0 | 20 | 0 | 20 | 0 | 20 | |
| **C2** | Power (W) | 60 | [C2 pulse repeated 10 times] | | | | | | | | |
| | Pulse duration (ms) | 0.17 | | | | | | | | | |
| | Energy (mJ) | 10 | | | | | | | | | |
| **D 2** | Power (W) | 1 | 60 | 0 | 60 | 0 | 60 | 0 | 60 | 0 | 60 |
| | Pulse duration (ms) | 50 | 0.17 | 50 | 0.17 | 50 | 0.17 | 50 | 0.17 | 50 | 0.17 |
| | Energy (mJ) | 50 | 10 | 0 | 10 | 0 | 10 | 0 | 10 | 0 | 10 |
| **E2** | Power (W) | 60 | 1 | 60 | 0 | 60 | 0 | 60 | 0 | 60 | |
| | Pulse duration (ms) | 0.17 | 50 | 0.17 | 50 | 0.17 | 50 | 0.17 | 50 | 0.17 | |
| | Energy (mJ) | 10 | 50 | 10 | 0 | 10 | 0 | 10 | 0 | 10 | |
| **F2** | Power (W) | 60 | 0 | 60 | 0 | 60 | 0 | 60 | 1 | 60 | |
| | Pulse duration (ms) | 017 | 50 | 017 | 50 | 0.17 | 50 | 0.17 | 50 | 017 | |
| | Energy (mJ) | 10 | 0 | 10 | 0 | 10 | 0 | 10 | 50 | 10 | |
| **G 2** | Power (W) | 60 | 0 | 60 | 0 | 60 | 0 | 60 | 0 | 60 | 1 |
| | Pulse duration (ms) | 0.17 | 50 | 0.17 | 50 | 0.17 | 50 | 0.17 | 50 | 0.17 | 50 |
| | Energy (mJ) | 10 | 0 | 10 | 0 | 10 | 0 | 10 | 0 | 10 | 50 |
| **H 2** | Power (W) | 60 | 1 | 60 | 1 | 60 | | 60 | 1 | 60 | |
| | Pulse duration (ms) | 0.17 | 12.5 | 0.17 | 12.5 | 0.17 | 12.5 | 0.17 | 12.5 | 0.17 | |
| | Energy (mJ) | 10 | 12.5 | 10 | 12.5 | 10 | 12.5 | 10 | 12.5 | 10 | |
| **I2** | Power (W) | 60 | | | | | | | | | |
| | Pulse duration (ms) | 0.83 | | | | | | | | | |
| | Energy (mJ) | 50 | | | | | | | | | |
| **J2** | Power (W) | 60 | 0 | 60 | 0 | 60 | 0 | 60 | 0 | 60 | |
| | Pulse duration (ms) | 0.17 | 50 | 0.17 | 50 | 0.17 | 50 | 0.17 | 50 | 0.17 | |
| | Energy (mJ) | 10 | 0 | 10 | 0 | 10 | 0 | 10 | 0 | 10 | |

It will thus be appreciated that those skilled in the art will be able to divise numerous systems, arrangements and methods which, although not explicitly shown or described herein, embody the principles of the present disclosure and are thus within the scope of the present disclosure.

## Claims

1. An apparatus for fractional resurfacing of skin tissue, comprising:
at least one electromagnetic radiation source arrangement;
an optical arrangement; and
a control arrangement,
wherein the at least one electromagnetic radiation source arrangement is configured to generate both ablative and non-ablative beams in the tissue,
wherein the control arrangement is configured to control the optical arrangement and the at least one source arrangement to generate and direct at least one first pulse of electromagnetic radiation, at least one second pulse of electromagnetic radiation, and at least one third pulse of electromagnetic radiation onto at least one single location on the skin tissue,
wherein each of the first, second and third pulses has a beam width that is less than about 1 mm,
wherein the control arrangement and the source arrangement are configured to provide the at least one first pulse, the at least one second pulse, and the at least one third pulse as successive pulses; and
wherein:
i. the at least one first pulse is an ablative pulse directed by the control arrangement to ablate at least one portion of the skin tissue to form a hole therein,
ii. the at least one second pulse is a non-ablative pulse directed by the control arrangement to coagulate tissue within the hole, and
iii. the at least one third pulse is an ablative pulse directed by the control arrangement to ablate at least one portion of the coagulated tissue within the hole.

2. The apparatus of claim 1, wherein the beam width of each of the first, second and third pulses is between about 0.1 mm and 0.5 mm.

3. The apparatus of claim 1, wherein the control arrangement is configured to direct at least one fourth pulse of electromagnetic radiation onto the at least one single location on the skin tissue, and to control properties of the at least one fourth pulse to coagulate further tissue within the hole.

4. The apparatus of any one of claims 1-3, wherein the at least one source arrangement comprises at least one of a CO2 laser, a CO laser, an erbium laser, an Er:YAG laser, an Er:YSGG laser, a Tm:YAG laser, an Ho:YAG laser, or a Nd:YAG laser.

5. The apparatus of claim 4, wherein the at least one source arrangement comprises a CO2 laser, and wherein a pulse energy of the at least one first pulse is between about 5 mJ and about 500 mJ.

6. The apparatus of claim 5, wherein a pulse duration of the at least one first pulse is between about 0.05 ms and about 5 ms.

7. The apparatus of claim 4, wherein the at least one source comprises a CO2 laser, and a pulse energy of the at least one second pulse is between about 5 mJ and about 500 mJ.

8. The apparatus of claim 7, wherein a pulse duration of the at least one second pulse is between about 0.5 ms and about 100 ms.

9. The apparatus of claim 4, wherein the at least one source comprises a CO2 laser, and a pulse energy of the at least one third pulse is between about 5 mJ and about 500 mJ.

10. The apparatus of claim 9, wherein a pulse duration of the at least one third pulse is between about 0.05 ms and about 5 ms.

11. The apparatus of claim 4, wherein the at least one source comprises a ER:YAG laser, and a pulse energy of the at least one first pulse is between about 100 mJ and about 1000 mJ.

12. The apparatus of claim 11, wherein a pulse duration of the at least one first pulse is between about 0.1 ms and about 20 ms.

13. The apparatus of claim 4, wherein the at least one source arrangement comprises a ER:YAG laser, and a pulse energy of the at least one second pulse is between about 10 mJ and about 500 mJ.

14. The apparatus of claim 13, wherein a pulse duration of the at least one second pulse is between about 5 ms and about 200 ms.

15. The apparatus of claim 4, wherein the at least one source arrangement comprises an ablative laser and a non-ablative laser,
wherein the control arrangement is configured to control the non-ablative laser to generate the at least one second pulse, and
wherein the control arrangement is further configured to control the ablative laser to generate at least one of the at least one first pulse or the at least one third pulse.

## Patentansprüche

1. Eine Vorrichtung zum fraktionalen Resurfacing von Hautgewebe, umfassend:
zumindest eine Quellenanordnung für elektromagnetische Strahlung; eine optische Anordnung; und eine Steuerungsanordnung,
wobei die zumindest eine Quellenanordnung für elektromagnetische Strahlung ausgebildet ist, ablative und nichtablative Strahlen im Gewebe zu erzeugen,
wobei die Steuerungsanordnung ausgebildet ist, die optische Anordnung und die zumindest eine Quellenanordnung zu steuern, um zumindest einen ersten Puls elektromagnetischer Strahlung, zumindest einen zweiten Puls elektromagnetischer Strahlung und zumindest einen dritten Puls elektromagnetischer Strahlung zu erzeugen und auf zumindest eine einzelne Stelle auf dem Hautgewebe auszurichten,
wobei jeder des ersten, zweiten und dritten Pulses einen Stahldurchmesser aufweist, welcher geringer ist als ungefähr 1 mm,
wobei die Steuerungsanordnung und die Quellenanordnung ausgebildet sind, den zumindest einen ersten Puls, den zumindest einen zweiten Puls und den zumindest einen dritten Puls als aufeinanderfolgende Pulse zu erzeugen; und
wobei:
i. der zumindest eine erste Puls ein ablativer Puls ist, welcher durch die Steuerungsanordnung zur Ablation zumindest eines Teils des Hautgewebes ausgerichtet ist, um ein Loch darin zu erzeugen,
ii. der zumindest eine zweite Puls ein nichtablativer Puls ist, welcher durch die Steuerungsanordnung ausgerichtet ist, um Gewebe innerhalb des Lochs zu koagulieren, und
iii. der zumindest eine dritte Puls ein ablativer Puls ist, welcher durch die Steuerungsanordnung ausgerichtet ist, um zumindest ein Teil des koagulierten Gewebes innerhalb des Lochs abzutragen.

2. Die Vorrichtung gemäß Anspruch 1, wobei der Strahldurchmesser von jedem des ersten, des zweiten und des dritten Pulses zwischen ungefähr 0,1 mm und 0,5 mm beträgt.

3. Die Vorrichtung gemäß Anspruch 1, wobei die Steuerungsanordnung ausgebildet ist, zumindest einen vierten Puls elektromagnetischer Strahlung auf die zumindest eine einzelne Stelle auf dem Hautgewebe zu richten und Eigenschaften des zumindest einen vierten Pulses zu steuern, um weiteres Gewebe innerhalb des Lochs zu koagulieren.

4. Die Vorrichtung gemäß einem der Ansprüche 1-3, wobei die zumindest eine Quellenanordnung zumindest eines aus dem Folgenden aufweist: einen CO2-Laser, einen CO-Laser, einen Erbium-Laser, einen Er:YAG-Laser, einen Er:YSGG Laser, einen Tm:YAG-Laser, einen Ho:YAG-Laser, oder einen Nd:YAG-Laser.

5. Die Vorrichtung gemäß Anspruch 4, wobei die zumindest eine Quellenanordnung einen CO2-Laser aufweist, und wobei eine Pulsenergie des zumindest einen ersten Pulses zwischen ungefähr 5 mJ und ungefähr 500 mJ beträgt.

6. Die Vorrichtung gemäß Anspruch 5, wobei eine Pulsdauer des zumindest einen ersten Pulses zwischen ungefähr 0,05 ms und ungefähr 5 ms beträgt.

7. Die Vorrichtung gemäß Anspruch 4, wobei die zumindest eine Quelle einen CO2-Laser umfasst und eine Pulsenergie des zumindest einen zweiten Pulses zwischen ungefähr 5 mJ und ungefähr 500 mJ beträgt.

8. Die Vorrichtung gemäß Anspruch 7, wobei eine Pulsdauer des zumindest einen zweiten Pulses zwischen ungefähr 0,5 ms und ungefähr 100 ms beträgt.

9. Die Vorrichtung gemäß Anspruch 4, wobei die zumindest eine Quelle einen CO2-Laser aufweist und eine Pulsenergie des zumindest einen dritten Pulses zwischen ungefähr 5 mJ und ungefähr 500 mJ beträgt.

10. Die Vorrichtung gemäß Anspruch 9, wobei eine Pulsdauer des zumindest einen dritten Pulses zwischen ungefähr 0,05 ms und ungefähr 5 ms beträgt.

11. Die Vorrichtung gemäß Anspruch 4, wobei die zumindest eine Quellenanordnung einen Er:YAG-Laser aufweist und eine Pulsenergie des zumindest einen ersten Pulses zwischen ungefähr 100 mJ und ungefähr 1000 mJ beträgt.

12. Die Vorrichtung gemäß Anspruch 11, wobei eine Pulsdauer des zumindest einen ersten Pulses zwischen ungefähr 0,1 ms und ungefähr 20 ms beträgt.

13. Die Vorrichtung gemäß Anspruch 4, wobei die zumindest eine Quellenvorrichtung einen ER:YAG-Laser aufweist und eine Pulsenergie des zumindest einen zweiten Pulses zwischen ungefähr 10 mJ ungefähr 500 mJ beträgt.

14. Die Vorrichtung gemäß Anspruch 13, wobei eine Pulsdauer des zumindest ein zweiten Pulse zwischen ungefähr 5 ms bis ungefähr 200 ms beträgt.

15. Die Vorrichtung gemäß Anspruch 4, wobei die zumindest eine Quellenanordnung einen ablativen Laser und einen nichtablativen Laser aufweist,
wobei die Steuerungsanordnung ausgebildet ist, den nichtablativen Laser zur Erzeugung des zumindest einen zweiten Pulses anzusteuern, und
wobei die Steuerungsanordnung ferner ausgebildet ist, den ablativen Laser zur Erzeugung zumindest eines des zumindest einen ersten Pulses oder des zumindest einen dritten Pulses anzusteuern.

## Revendications

1. Un appareil de resurfaçage fractionné de tissu cutané comprenant :
au moins un agencement formant source de rayonnement électromagnétique ;
un agencement optique ; et
un agencement de commande,
ledit au moins un agencement formant source de rayonnement électromagnétique étant configuré pour générer à la fois des rayons ablatifs et non ablatifs dans le tissu,
l'agencement de commande étant configuré pour commander l'agencement optique et ledit au moins un agencement formant source de façon à générer et diriger au moins une première impulsion de rayonnement électromagnétique, au moins une deuxième impulsion de rayonnement électromagnétique et au moins une troisième impulsion de rayonnement électromagnétique sur au moins un emplacement unique sur le tissu cutané,
chacune des première, deuxième et troisième impulsions ayant une largeur de faisceau inférieure à environ 1 mm,
l'agencement de commande et l'agencement formant source sont configurés pour fournir ladite au moins une première impulsion, ladite au moins une deuxième impulsion et ladite au moins une troisième impulsion en tant qu'impulsions successives ; et
i. ladite au moins une première impulsion étant une impulsion ablative dirigée par l'agencement de commande pour ablater au moins une partie du tissu cutané afin d'y former un trou,
ii. ladite au moins une deuxième impulsion étant une impulsion non ablative dirigée par l'agencement de commande pour coaguler le tissu dans le trou, et
iii. ladite au moins une troisième impulsion étant une impulsion ablative dirigée par l'agencement de commande pour ablater au moins une partie du tissu coagulé dans le trou.

2. L'appareil selon la revendication 1, dans lequel la largeur de faisceau de chacune des première, deuxième et troisième impulsions est comprise entre environ 0,1 mm et 0,5 mm.

3. L'appareil selon la revendication 1, dans lequel l'agencement de commande est configuré pour diriger au moins une quatrième impulsion de rayonnement électromagnétique sur ledit au moins un emplacement unique sur le tissu cutané, et pour commander les propriétés de ladite au moins une quatrième impulsion de façon à coaguler du tissu supplémentaire dans le trou.

4. L'appareil selon l'une quelconque des revendications 1 à 3, dans lequel ledit au moins un agencement formant source comprend au moins l'un parmi : un laser au CO2, un laser au CO, un laser erbium, un laser Er:YAG, un laser Er:YSGG, un laser Tm:YAG, un laser Ho:YAG ou un laser Nd:YAG.

5. L'appareil selon la revendication 4, dans lequel ledit au moins un agencement formant source comprend un laser à CO2, et dans lequel une énergie d'impulsion de ladite au moins une première impulsion est comprise entre environ 5 mJ et environ 500 mJ.

6. L'appareil selon la revendication 5, dans lequel une durée d'impulsion de ladite au moins une première impulsion est comprise entre environ 0,05 ms et environ 5 ms.

7. L'appareil selon la revendication 4, dans lequel ladite au moins une source comprend un laser au CO2, et une énergie d'impulsion de ladite au moins une deuxième impulsion est comprise entre environ 5 mJ et environ 500 mJ.

8. L'appareil selon la revendication 7, dans lequel une durée d'impulsion de ladite au moins une deuxième impulsion est comprise entre environ 0,5 ms et environ 100 ms.

9. L'appareil selon la revendication 4, dans lequel ladite au moins une source comprend un laser au CO2, et une énergie d'impulsion de ladite au moins une troisième impulsion est comprise entre environ 5 mJ et environ 500 mJ.

10. L'appareil selon la revendication 9, dans lequel une durée d'impulsion de ladite au moins une troisième impulsion est comprise entre environ 0,05 ms et environ 5 ms.

11. L'appareil selon la revendication 4, dans lequel ladite au moins une source comprend un laser ER:YAG, et une énergie d'impulsion de ladite au moins une première impulsion est comprise entre environ 100 mJ et environ 1000 mJ.

12. L'appareil selon la revendication 11, dans lequel une durée d'impulsion de ladite au moins une première impulsion est comprise entre environ 0,1 ms et environ 20 ms.

13. L'appareil selon la revendication 4, dans lequel ledit au moins un agencement formant source comprend un laser ER:YAG, et une énergie d'impulsion de ladite au moins une deuxième impulsion est comprise entre environ 10 mJ et environ 500 mJ.

14. L'appareil selon la revendication 13, dans lequel une durée d'impulsion de ladite au moins une deuxième impulsion est comprise entre environ 5 ms et environ 200 ms.

15. L'appareil selon la revendication 4, dans lequel ledit au moins un agencement formant source comprend un laser ablatif et un laser non ablatif,
l'agencement de commande étant configuré pour commander le laser non ablatif de façon à générer ladite au moins une deuxième impulsion, et l'agencement de commande étant en outre configuré pour commander le laser ablatif de façon à générer au moins une parmi : ladite au moins une première impulsion et ladite au moins une troisième impulsion.
